(19) Europäisches Patentamt
European Patent Office
Office européen des brevets

(11) Numéro de publication: **0 320 369 B1**

(12) # FASCICULE DE BREVET EUROPEEN

(45) Date de publication de fascicule du brevet: **14.04.93**

(51) Int. Cl.[5]: **C07D 403/14**, C06B 25/04

(21) Numéro de dépôt: **88403088.3**

(22) Date de dépôt: **06.12.88**

(54) **5-Nitro-4,6-bis-(3-nitro-1H-1,2,4-triazol-1-yl)pyrimidine, son procédé de préparation et matériau explosif la contenant.**

(30) Priorité: **08.12.87 FR 8717060**

(43) Date de publication de la demande:
**14.06.89 Bulletin 89/24**

(45) Mention de la délivrance du brevet:
**14.04.93 Bulletin 93/15**

(84) Etats contractants désignés:
**CH DE GB IT LI SE**

(56) Documents cités:
**US-A- 2 987 520**
**US-A- 3 483 211**
**US-A- 3 923 804**

(73) Titulaire: **COMMISSARIAT A L'ENERGIE ATOMIQUE**
**31/33, rue de la Fédération**
**F-75015 Paris(FR)**

(72) Inventeur: **Freche, Jean-Paul**
**11 rue du Grand Rabbin**
**F-54000 Nancy(FR)**
Inventeur: **Laval, François**
**Avenue Joliot Curie**
**F-37260 Monts(FR)**
Inventeur: **Wartenberg, Christian**
**12, rue du Bois d'Azay**
**F-37260 Monts(FR)**

(74) Mandataire: **Mongrédien, André et al**
**c/o BREVATOME 25, rue de Ponthieu**
**F-75008 Paris (FR)**

**EP 0 320 369 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet un nouveau dérivé de pyrimidine, son procédé de fabrication et son utilisation comme explosif.

De façon plus précise, elle concerne un nouveau dérivé de pyrimidine utilisable comme explosif secondaire, pour l'équipement de missiles et armements modernes.

Pour ces applications, il est souvent intéressant d'utiliser des explosifs ayant une sensibilité au choc aussi faible que possible, mais une puissance élevée, c'est-à-dire la capacité de délivrer une énergie très élevée. Ces deux propriétés sont difficiles à trouver simultanément dans un même explosif. Ainsi, le triaminotrinitrobenzène (TATB) est très peu sensible au choc, mais il manque de puissance, alors que la cyclotétraméthylène tétranitramine (octogène) qui est très puissante, est plus sensible au choc et aux agressions.

Aussi, des recherches ont été effectuées récemment pour mettre au point de nouveaux explosifs dont la sensibilité au choc se rapproche de celle du TATB tout en étant capables de délivrer une énergie plus élevée que ce dernier, se rapprochant de celle de l'octogène.

Le document US-A- 3 923 804 décrit des composés explosifs résistant à la chaleur de formule :

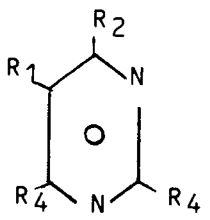

dans laquelle $R_1$ est H ou $NO_2$ et $R_2$, $R_3$ et $R_4$ sont H ou

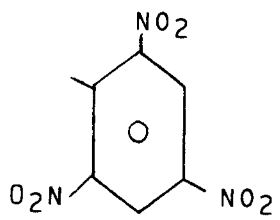

par exemple le 4,6-dipicryl-5-nitropyrimidine.

La présente invention a pour objet un autre dérivé de pyrimidine présentant les propriétés recherchées, décrites ci-dessus, c'est-à-dire une sensibilité au choc proche de celle du TATB, tout en étant capable de délivrer une énergie plus élevée.

Le nouveau dérivé de pyrimidine de l'invention est la 4,6-di-2(5-nitro-1,2,4-triazole)-5-nitropyrimidine répondant à la formule :

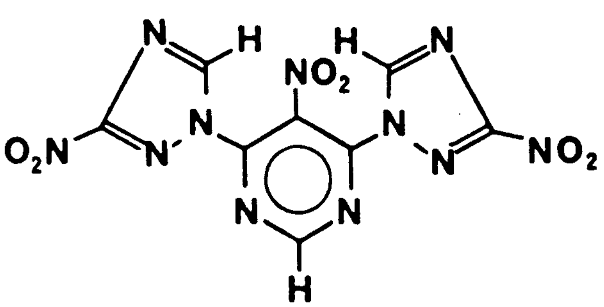

(I)

Ce nouveau dérivé de pyrimidine présente un grand intérêt pour une utilisation comme explosif secondaire car ses propriétés détoniques sont intermédiaires entre celles du TATB et celles de l'octogène, en ce qui concerne la sensibilité au choc et la vitesse de détonation.

Ce nouveau dérivé de pyrimidine peut être préparé par un procédé consistant à faire réagir un 4,6-dihalogéno-5-nitro-pyrimidine de formule :

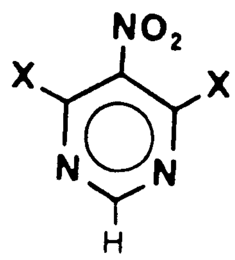

(II)

dans laquelle X représente le fluor, le chlore ou le brome, avec le 5-nitro-1,2,4-triazole de formule :

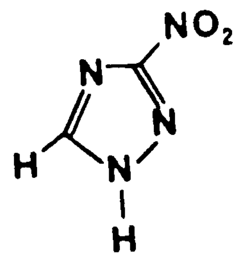

(III)

Ce procédé est basé sur une réaction de substitution nucléophile entre le 5-nitro-1,2,4-triazole de formule III et une 4,6-dihalogéno-5-nitropyrimidine de formule II qui sont des réactifs de départ disponibles sur le marché ou de synthèse aisée. La réaction est simple et rapide, elle correspond au schéma réactionnel suivant :

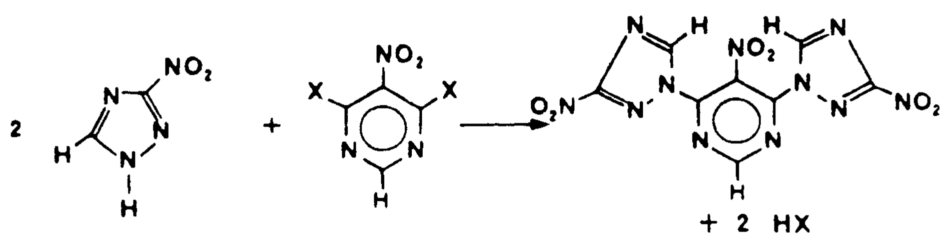

(III) (II) (I)

Le 5-nitro-1,2,4-triazole de formule III utilisé comme produit de départ pour cette réaction est un produit disponible commercialement. On peut aussi le préparer aisément au laboratoire à partir du 5-amino-1,2,4-triazole qui est un composé fabriqué en grande quantité par les industries phytosanitaires et photographiques. Une méthode couramment utilisée consiste à effectuer une diazotation par action de l'acide nitreux puis une substitution du groupe diazonium par l'ion nitrite $NO_2^-$. En effet, le caractère aromatique du cycle 1,2,4-triazole permet la diazotation par action de l'acide nitreux. La substitution du groupe diazonium ainsi formé par l'ion nitrite peut ensuite se faire sous l'action d'une solution aqueuse de nitrite de sodium, selon le schéma réactionnel suivant :

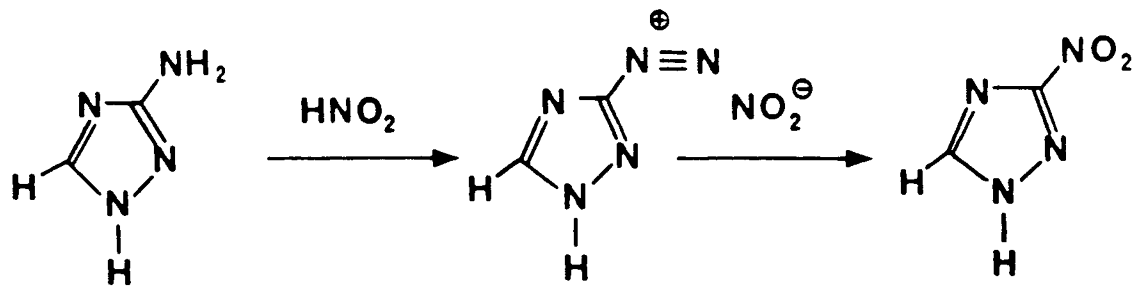


De nombreux modes opératoires basés sur ces réactions ont été décrits en particulier par L.I. BAGAL et al. dans Chemistry of heterocyclic compounds, 6, 265 (1970), M.S. PEVZNER et al dans Khimiya Getero Soedin, 8, 1132 (1979), J.W. JONES et al dans J. Al. Chem. Soc. 82, 3773 (1960), E.J. BROWNE dans Aust. J. Chem. 22, 2251 (1969) et J.L. CLOSSET et al dans Bull. Soc. Chim. Belge. 84 (1975).

L'halogène des 4,6-dihalogéno-5-nitropyrimidines peut être le fluor, le chlore ou le brome.

De préférence, on utilise dans le procédé de l'invention la 4,6-dichloro-5-nitropyrimidine, en raison de sa bonne disponibilité commerciale et de la grande réactivité de ses deux atomes de chlore.

La réaction de substitution des deux atomes d'halogène de la pyrimidine par les deux cycles triazole est très rapide en raison de l'effet mésomère électro-attracteur très puissant du groupement nitro. Cette réaction peut donc être effectuée à la température ambiante, de préférence en présence d'un composé capable de piéger l'acide chlorhydrique formé au cours de la réaction. Ce composé peut être en particulier une amine tertiaire telle que la triéthylamine.

Pour réaliser la réaction, on utilise généralement un solvant des produits de départ dans lequel la 4,6-di-2(5-nitro1,2,4-triazole)-5 nitro-pyrimidine est insoluble. Ce solvant peut être par exemple un alcool tel que le propanol-2.

La réaction entre la 4,6-dihalogéno-5-nitro-pyrimidine et le 5-nitro-1,2,4-triazole conduit à un mélange de deux isomères qui se différencient par la présence d'une ou de deux liaisons hydrogènes intramoléculaires entre le groupement nitro central et les deux atomes d'hydrogène des cycles triazoles. Ces deux isomères répondent aux formules suivantes :

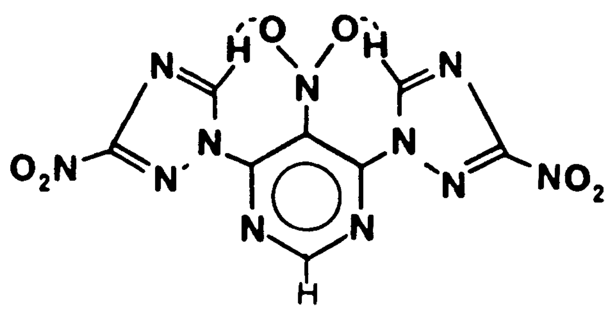

$(I_a)$

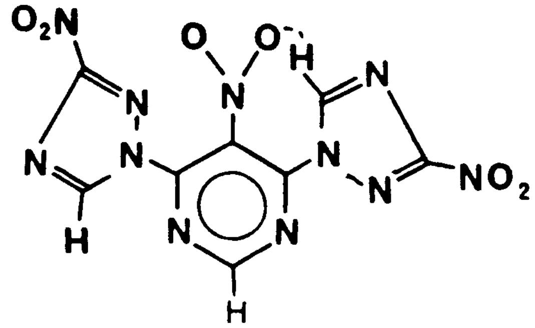

$(I_b)$

L'isomère de formule $I_b$ qui est thermodynamiquement moins stable, est très minoritaire. Ainsi, il suffit de recristalliser dans l'acétonitrile le produit obtenu par la réaction entre les composés de formule II et III pour faire disparaître l'isomère de formule $I_b$ au profit de l'isomère de formule $I_a$.

Le dérivé de pyrimidine de l'invention peut être utilisé comme matériau explosif. Dans ce cas, ce dérivé est généralement dispersé dans un liant thermoplastique ou thermoducissable contenant éventuellement d'autres additifs habituellement utilisés dans de telles compositions (plastifiants, etc).

L'exemple suivant illustre la préparation de la 4,6-di-2(5-nitro-1,2,4-triazole)-5-nitropyrimidine à partir de la 4,6-dichloro-5-nitropyrimidine du commerce et du 5-nitro-1,2,4-triazole que l'on prépare à partir du 5-amino-1,2,4-triazole.

I. Préparation du 5-nitro-1,2,4-triazole.

On dissout 1,68g (0,02 mole) de 5-amino-1,2,4-triazole dans 16 ml d'acide acétique glacial et on ajoute la solution obtenue à une solution constituée de 7 ml d'$H_2SO_4$ concentré et 1,6g (0,023 mole) de nitrite de sodium $NaNO_2$.

On maintient le mélange à une température allant de 0 à 5°C. Après 5 min, on ajoute 50 ml d'eau tout en maintenant la température au voisinage de 0°C.

On ajoute la solution obtenue à 200 ml de nitrite de sodium à 10% chauffé à 45-50°C. Après une heure à 45°C, on acidifie la solution avec 0,6 ml d'$H_2SO_4$ puis on la traite avec 1,2g d'urée.

On extrait ensuite le 5-nitro-1,2,4-triazole par de l'acétate d'éthyle et on le recristallise dans du méthanol. Son point de fusion est de 210°C.

Le rendement de la réaction est de 57%.

II. Préparation de la 4,6-di-2(5-nitro-1,2,4-triazole)-5-nitropyrimidine.

On dissout $5.10^{-3}$ mole de 4,6-dichloro-5-nitro-pyrimidine dans 15 ml de propanol-2 et on chauffe à reflux.

On dissout $1,1.10^{-3}$ mole de 3-nitro-1,2,4-triazole dans 25 ml de propanol-2 et 1,6 ml de triéthylamine et on ajoute ce mélange à la solution de dichloronitropyrimidine.

Il se forme très rapidement un précipité que l'on filtre et lave abondamment au propanol-2 et à l'eau.

On purifie ensuite ce produit par solubilisation dans l'acétone suivie d'une reprécipitation à l'eau. On filtre le produit et on le recristallise dans l'acétonitrile.

Le rendement est de 74%.

L'analyse élémentaire du produit obtenu est la suivante :

| | C | H | N |
|---|---|---|---|
| Trouvée | 27,66 | 0,91 | 44,15 |
| Calculée | 27,52 | 0,87 | 44,12 |

Le produit obtenu se présente sous l'aspect d'un solide blanc cristallisé dont le point de fusion est à 248°C.

Sa densité, mesurée au picnomètre à gaz, est P = 1,74.

Il est soluble dans l'acétone, la méthyléthylcétone, l'acétate d'éthyle, l'acétonitrile, le diméthylacétamide, le diméthylformamide (DMF), le diméthylsulfoxyde (DMSO), et le nitrobenzène.

Il est insoluble dans l'eau, les alcools, les solvants chlorés, le benzène.

Sa surface spécifique est de 2,70 $m^2/g$ (mesurée par la méthode BET).

Il présente les propriétés suivantes :

I. Propriétés spectroscopiques

1) Résonance magnétique Nucléaire (RMN)

En adoptant pour les différents noyaux de la molécule les notations suivantes :

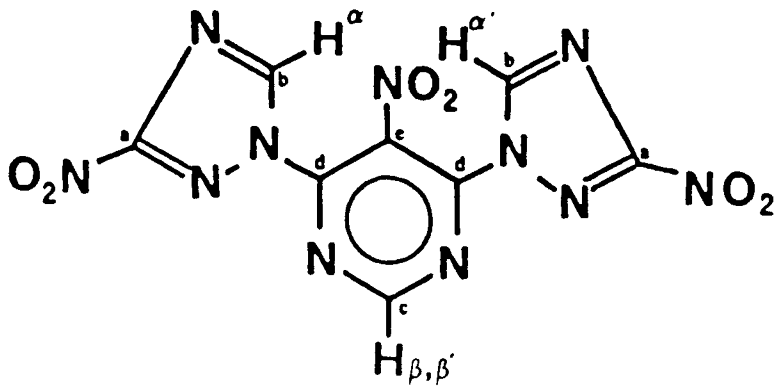

les résultats obtenus en RMN sont les suivants :

a) RMN du proton $^1H$

Le spectre proton $^1H$ à 60 MHz de l'échantillon en solution dans le diméthylsulfoxyde (DMSO $d_6$) comporte deux pics :

- 1 pic à 10,00 ppm correspondant aux protons H($\alpha$) du cycle triazole engagés dans une liaison hydrogène avec le groupement nitro,
- 1 pic à 9,46 ppm dû au proton H($\beta$) en 2, du cycle pyrimidine.

Ces pics sont caractéristiques de l'isomère (A) comportant deux liaisons hydrogène intramoléculaires.

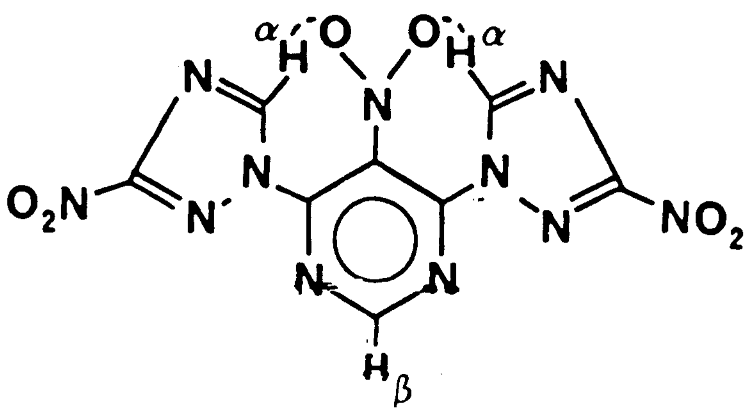
(A)

Dans le cas d'un échantillon non recristallisé dans l'acétonitrile, la présence d'un faible pourcentage de l'isomère (B) ne comportant qu'une seule liaison hydrogène intramoléculaire, se traduit par l'apparition de deux petits pics supplémentaires :

9,54 ppm H($\beta$') du cycle pyrimidine,

8,96 ppm H($\alpha$') du cycle triazole non engagé dans une liaison hydrogène.

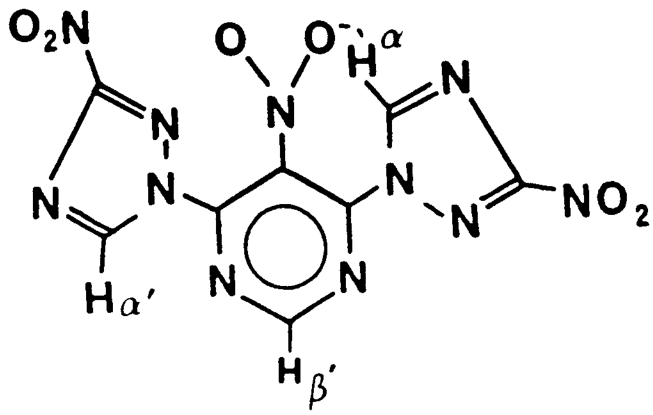

(B)

b) RMN du carbone $^{13}C$

Le spectre à 20,15 MHz de l'échantillon dissout dans l'acétone $d_6$ en présence d'un agent de relaxation constitué par l'acétylacétonate de chrome donne les déplacements chimiques suivants :

| δ (ppm) | Attribution | | |
|---|---|---|---|
| 165,20 | $C_{(a)}$ | C - $NO_2$ | (triazole) |
| 159,20 | $C_{(c)}$ | C - H | (pyrimidine) |
| 148,51 | $C_{(b)}$ | C - H | (triazole) |
| 148,33 | $C_{(d)}$ | C - NTr (Tr encerclé) | (pyrimidine) |
| 125,31 | $C_{(e)}$ | C - $NO_2$ | (pyrimidine) |

Le spectre infrarouge fait apparaître un ensemble de bandes difficilement attribuables indiquant la présence de cycles aromatiques nitrés.

La bande C-H du triazole apparaît à 3150 $cm^{-1}$.

II. Propriétés thermique et détoniques

1) Balance en oxygène.

La balance en oxygène par rapport au $CO_2$ et $H_2O$ est de -55,78g d'$O_2$ pour 100g du produit. A titre comparatif, la balance en oxygène du TATB est de -55,81g/100g et celle de l'octogène de -21,6 g/100g.

2) Température de déflagration.

Un échantillon d'environ 20 mg en conteneur d'acier inoxydable est trempé dans un bain dont la température est élevée à 5 ° C/min. Dans le cas présent, la température à laquelle le produit déflagre est de 356 ° C.

3) Temps d'induction thermique.

On introduit brusquement dans un bain à la température de mesure environ 10mg du produit dans un conteneur d'acier. On enregistre le temps au bout duquel on a décomposition. En considérant que la cinétique est d'ordre zéro, on peut calculer l'énergie d'activation.

Pour le 4,6-di-2(5-nitro-1,2,4-triazole)-5-nitro-pyrimidine, la déflagration se produit au bout de 5s pour une température de 380 ° C.

L'énergie d'activation de la réaction est de 39,7 kcal/mole.

4) Vitesse de détonation.

La vitesse de détonation calculée à partir de la formule selon la méthode empirique de Rothstein et Petersen décrite dans Propellants and Explosives 4, 56-60 (1979) est, pour la densité de cristal, de 8420 m/s.

A titre comparatif, selon cette même méthode, la vitesse de détonation calculée du TATB est de 7870 m/s et celle de l'octogène de 9050 m/s.

5) Sensibilité au choc.

La sensibilité au choc du produit a été déterminée à l'aide d'un mouton pendulaire de 5kg, un échantillon de 30 mg étant déposé sur papier de verre. La conduite de l'essai se fait selon la méthode de Bruceton.

Dans de telles conditions d'essai, aucune réaction pyrotechnique n'a été obtenue pour la hauteur maximale du pendule.

H > 72 cm

A titre comparatif, la sensibilité au choc du TATB correspond à $H_{50}>72$ cm et celle de l'octogène à un $H_{50}$ de 15 cm.

On constate ainsi que la 4,6-di-2-(5-nitro-1,2,4-triazole)-5-nitropyrimidine a une sensibilité au choc très faible tout en étant capable de délivrer une énergie élevée.

## Revendications

**1.** 4,6-di-2-(5-nitro-1,2,4-triazole)-5-nitropyrimidine de formule :

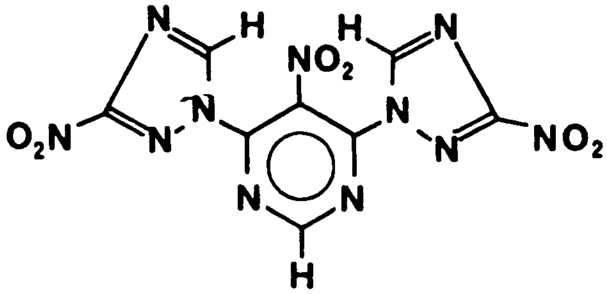

(I)

sous forme d'isomère pur ou de mélanges d'isomères.

**2.** 4,6-di-2-(5-nitro-1,2,4-triazole)-5-nitropyrimidine selon la revendication 1 sous la forme d'isomère de formule :

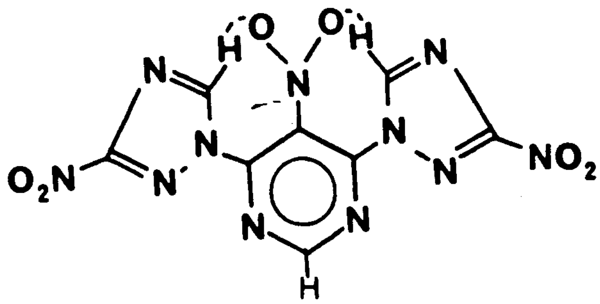

(I a)

3. Procédé de préparation de la 4,6-di-2-(5-nitro-1,2,4-triazole)-5-nitropyrimidine de formule (I) de la revendication 1 caractérisé en ce qu'il consiste à faire réagir un 4,6-dihalogéno-5-nitropyrimidine de formule :

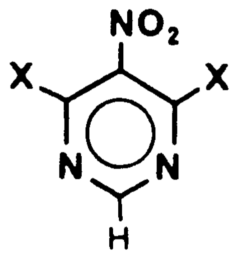

(II)

dans laquelle X représente le fluor, le chlore ou le brome, avec le 5-nitro-1,2,4-triazole de formule :

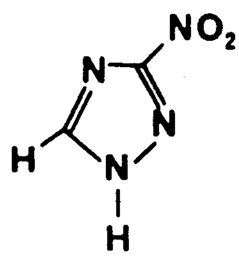

(III)

4. Procédé selon la revendication 3, caractérisé en ce que l'on recristallise dans l'acétonitrile le produit obtenu par réaction du 5-nitro-1,2,4-triazole et de la 4,6-dihalogéno-5-nitropyrimidine pour obtenir l'isomère de formule :

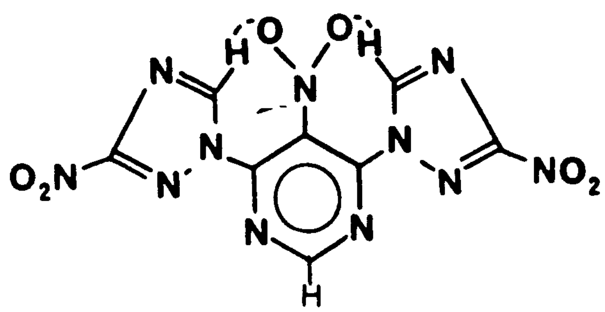

(I a)

5. Matériau explosif, caractérisé en ce qu'il comprend un isomère ou un mélange d'isomères de la 4,6-di-2-(5-nitro-1,2,4-triazole)-5-nitropyrimidine de formule (I) ou (Ia) selon la revendication 1 ou 2.

## Claims

**1.** 4,6-di-2-(5-nitro-1,2,4-triazole)-5-nitropyrimidine of formula:

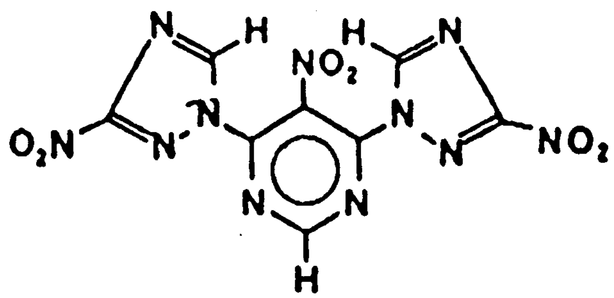

(I)

in the form a pure isomer or mixtures of isomers.

**2.** 4,6-di-2-(5-nitro-1,2,4-triazole)-5-nitropyrimidine of formula:

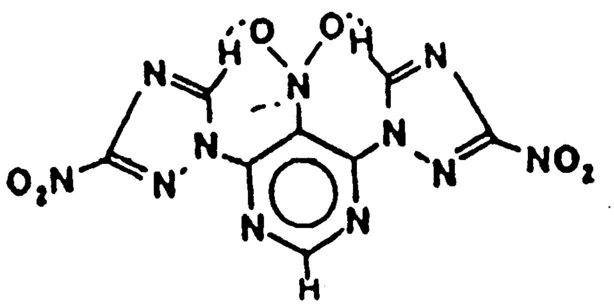

($I_a$)

**3.** Process for the preparation of 4,6-di-2-(5-nitro-1,2,4-triazole)-5-nitropyrimidine of formula (I) of claim 1, characterized in that it comprises reacting a 4,6-dihalogeno-5-nitropyrimidine of formula:

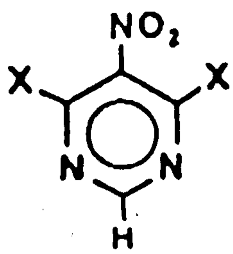

(II)

in which X represents fluorine, chlorine or bromine, with 5-nitro-1,2,4-triazole of formula:

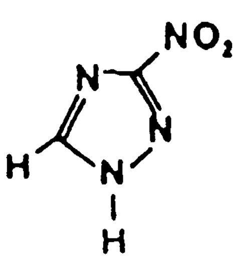

(III)

4. Process according to claim 3, characterized in that recrystallization takes place in acetonitrile of the product obtained by the reaction of 5-nitro-1,2,4-triazole and 4,6-dihalogeno-5-nitropyrimidine in order to obtain the isomer of formula:

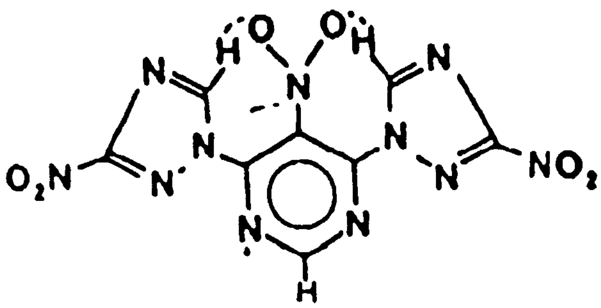

(Ia)

5. Explosive material, characterized in that it comprises an isomer or a mixture of isomers of 4,6-di-2-(5-nitro-1,2,4-triazole)-5-nitropyrimidine of formula (I) or (Ia) according to claims 1 or 2.

**Patentansprüche**

1. 4,6-Di-2-(5-nitro-1,2,4-triazol)-5-nitropyrimidin der Formel:

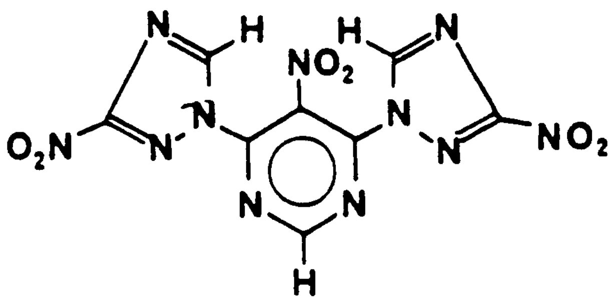

(I)

in der Form eines reinen Isomers oder von Isomerengemischen.

2. 4,6,-Di-2-(5-nitro-1,2,4-triazol)-5-nitropyrimidin nach Anspruch 1 in der Form des Isomers der Formel:

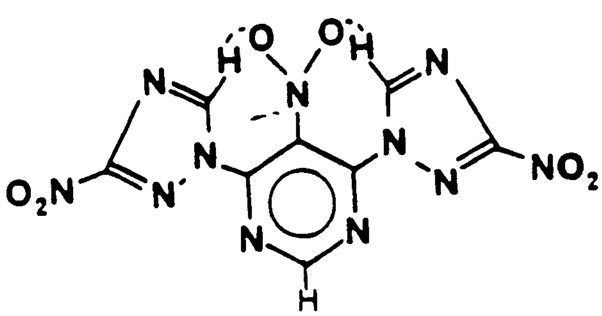

(Ia)

3. Verfahren zur Herstellung des 4,6-Di-2-(5-nitro-1,2,4-triazol)-5-nitropyrimidins der Formel (I) von Anspruch 1, dadurch gekennzeichnet, daß es das Umsetzen eines 4,6-Dihalogen-5-nitropyrimidins der Formel:

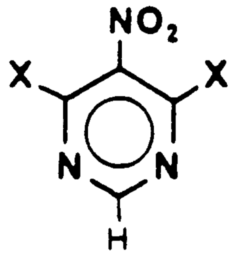

(II)

worin X Fluor, Chlor oder Brom bedeutet, mit dem 5-Nitro-1,2,4-triazol der Formel:

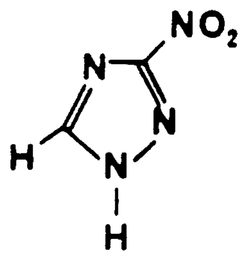

(III)

umfaßt.

**4.** Verfahren nach Anspruch 3, dadurch gekennzeichnet, daß das durch die Reaktion des 5-Nitro-1,2,4-triazols und des 4,6-Dihalogen-5-nitropyrimidins erhaltene Produkt in Acetonitril umkristallisiert wird, um das Isomer der Formel:

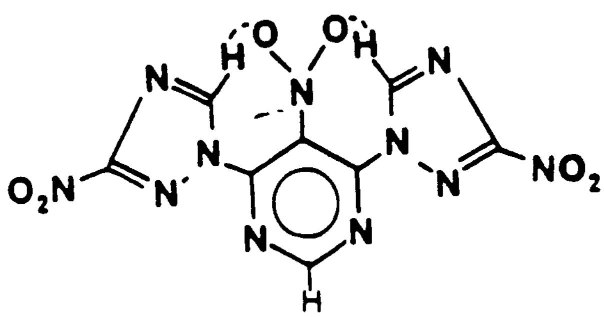

($I_a$)

zu erhalten.

**5.** Sprengstoff, dadurch gekennzeichnet, daß er ein Isomer oder ein Isomerengemisch von 4,6-Di-2-(5-nitro-1,2,4-triazol)-5-nitropyrimidin der Formel (I) oder (Ia) nach Anspruch 1 oder 2 umfaßt.